(19) Europäisches Patentamt
European Patent Office
Office européen des brevets

(11) EP 3 744 848 A1

(12) **EUROPEAN PATENT APPLICATION**
published in accordance with Art. 153(4) EPC

(43) Date of publication:
**02.12.2020 Bulletin 2020/49**

(21) Application number: **19743233.9**

(22) Date of filing: **25.01.2019**

(51) Int Cl.:
***C12N 15/70*** (2006.01) ***C07K 14/575*** (2006.01)
***C07K 7/08*** (2006.01) ***C07K 14/00*** (2006.01)

(86) International application number:
**PCT/KR2019/001055**

(87) International publication number:
**WO 2019/147055 (01.08.2019 Gazette 2019/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.01.2018 KR 20180010055**

(71) Applicant: **Huons Co., Ltd.**
**Seongnam-si, Gyeonggi-do 13486 (KR)**

(72) Inventors:
- **KIM, Yeong-Mok**
**Seoul 04732 (KR)**
- **KIM, Wanseop**
**Yongin-si, Gyeonggi-do 16822 (KR)**
- **UM, Key-An**
**Suwon-si, Gyeonggi-do 16295 (KR)**

(74) Representative: **Dehns**
**St. Bride's House**
**10 Salisbury Square**
**London EC4Y 8JD (GB)**

(54) **RECOMBINANT VECTOR CARRYING DISULFIDE BOND ISOMERASE SIGNAL PEPTIDE AND USE THEREOF**

(57) The present invention relates to a recombinant vector carrying a disulfide bond isomerase signal peptide and a use thereof. More particularly, the use of the recombinant vector of the present invention is advantageous in that a protein whose amino acid sequence does not start with methionine can be produced through an E. coli expression system. Thus, the present invention does not need additional processes for commercial availability, such as glycosylation and the like, and thus is expected to find useful applications in various purposes such as the development of therapeutic agents, etc. in the future.

FIG. 2

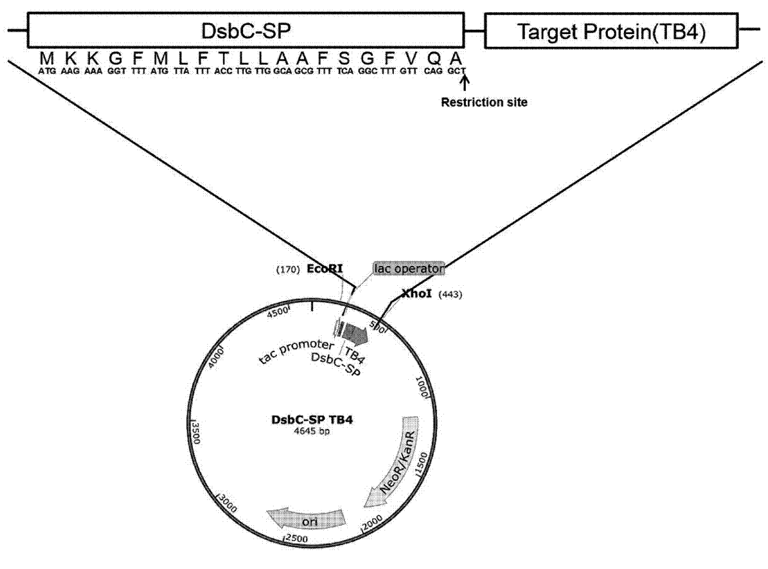


EP 3 744 848 A1

## Description

[Technical Field]

The present invention relates to a recombinant vector carrying a disulfide bond isomerase signal peptide and a use thereof.

This application claims priority to and the benefit of Korean Patent Application No. 10-2018-0010055 filed in the Korean Intellectual Property Office on January 26, 2018, and all the contents disclosed in the specification and drawings of that application are incorporated in this application.

[Background Art]

In order to mass-produce recombinant proteins for various purposes such as research, treatment, or other commercial purposes, various vectors and hosts are currently used, and among them, a method for expressing a required protein using an *E. coli* expression system, and then purifying the protein has been usefully utilized because a large amount of protein can be obtained with less cost and effort.

However, since there is a difference in intracellular environment between mammals and bacteria, when efforts are made to obtain various types of human proteins using an *E. coli* system, specific expression and purification conditions for each protein need to be established. In particular, amino acid sequences of all proteins produced from *E. coli* start with methionine (Met), and in contrast, proteins found in the human body or expressed from advanced organisms are degraded by proteases through post-translational modification and activated or subjected to various protein modifications such as glycosylation which attach sugar chains, phosphorylation, acetylation, and carboxylation. Therefore, amino acid sequences of many commercialized proteins do not start with methionine, and accordingly, proteins produced from *E. coli* are disadvantageous in that the proteins need to be subjected to an additional process such as proteolytic cleavage by enzymatic reactions.

Meanwhile, thymosin beta 4 (TB4) is known to exert efficacy in neovascularization, wound healing, hair growth, and the like, and thus is currently experiencing clinical trials and FDA approval reviews for use as a therapeutic agent for various diseases such as ischemic heart disease, corneal injury, ophthalmic diseases, and epidermolysis bullosa, and is expected to be developed as a therapeutic agent for alopecia.

Since thymosin beta 4 is highly available as a therapeutic agent for various diseases as described above, it is expected that there will be huge demand when thymosin beta 4 is released as a therapeutic peptide in the future, but there are problems in that when the peptide is produced by synthesis, high production costs are required, and when the peptide is expressed from a host (host strain) such as *E. coli,* additional work such as cleavage by enzymatic reactions after production in order to modify a protein sequence which starts with methionine is required, but research on this is still insufficient.

[Disclosure]

[Technical Problem]

The present inventors have made intensive efforts to solve the problem in the related art as described above, and as a result, confirmed that when a recombinant vector comprising a disulfide bond isomerase signal peptide was used, a protein whose desired amino acid sequence does not start with methionine could be produced through an *E. coli* expression system, thereby completing the present invention based on this.

Thus, an object of the present invention is to provide a recombinant vector comprising a base sequence encoding a disulfide bond isomerase signal peptide.

Further, another object of the present invention is to provide a recombinant vector comprising base sequences encoding a disulfide bond isomerase signal peptide and thymosin beta 4.

In addition, still another object of the present invention is to provide a transformant transformed with the recombinant vector.

Furthermore, yet another object of the present invention is to provide a method for producing a thymosin beta 4 protein, the method comprising: transforming *E. coli* with the recombinant vector, and then culturing the *E. coli* in a medium.

However, technical problems to be achieved by the present invention are not limited to the aforementioned problems, and other problems that are not mentioned may be clearly understood by those skilled in the art from the following description.

[Technical Solution]

The present invention relates to a recombinant vector comprising a disulfide bond isomerase signal peptide and a use thereof, and the present invention provides a recombinant vector comprising a base sequence encoding a disulfide bond isomerase signal peptide.

Further, the present invention provides a recombinant vector comprising base sequences encoding a disulfide bond isomerase signal peptide and thymosin beta 4.

In addition, the present invention provides a transformant transformed with the recombinant vector.

Furthermore, the present invention provides a method for producing a thymosin beta 4 protein, the method comprising: transforming *E. coli* with the recombinant vector, and then culturing the *E. coli* in a medium.

[Advantageous Effects]

When a recombinant vector comprising a disulfide bond isomerase signal peptide of the present invention is used, it is possible to produce a protein whose amino acid sequence does not start with methionine through an *E. coli* expression vector, and accordingly, there is an advantage in that the recombinant vector does not need to be subjected to an additional process such as a protease treatment to be commercially used. In particular, although thymosin beta 4 has been highly available as a therapeutic agent for various diseases for a while, thymosin beta 4 has a problem in that when thymosin beta 4 is expressed in a host (host strain), thymosin beta 4 is degraded by intracellular proteases and cannot be produced. The present invention enables the thymosin beta 4 to be produced through an *E. coli* expression system, and thus to be usefully utilized in the development of a therapeutic agent for alopecia as well as ischemic heart disease, corneal injury, ophthalmic diseases, and epidermolysis bullosa, and further, the *E. coli* expression system of the present invention can be applied to production of various therapeutic proteins such as a parathyroid hormone, a human growth hormone, and a granulocyte-colony stimulating factor (GCSF) used for the treatment of osteoporosis, and thus is expected to be widely used as a useful technique.

[Description of Drawings]

FIG. 1 schematically illustrates the result of confirming the alignment of a DNA sequence of a synthesized plasmid DsbC-SP TB4 and a reference DNA sequence.

FIG. 2 illustrates a vector map of DsbC-SP TB4.

FIG. 3 schematically illustrates the growth degree of a transformed *E. coli* strain BL21.

FIG. 4 schematically illustrates the result of inducing the expression of a thymosin beta 4 (TB4) protein with 1.0 mM IPTG.

FIG. 5 illustrates the result of confirming the band of a transformed target protein with SDS-PAGE.

[Modes of the Invention]

The present inventors confirmed that when a recombinant vector comprising a disulfide bond isomerase signal peptide was used, a protein whose amino acid sequence does not start with methionine could be produced through an *E. coli* expression system, thereby completing the present invention based on this.

Hereinafter, the present invention will be described in detail.

The present invention provides a recombinant vector comprising a base sequence encoding a disulfide bond isomerase signal peptide.

Further, the present invention provides a recombinant vector comprising a base sequence of SEQ ID NO: 7 in which thymosin beta 4 is linked to the carboxyl terminus of the disulfide bond isomerase signal peptide.

As used herein, the "vector" refers to DNA capable of being proliferated by introducing a target DNA fragment into a host bacterium and the like in a DNA recombination experiment, and is also called a cloning vehicle, wherein vector DNA is cleaved by a restriction enzyme and the like to open the ring, and the target DNA fragment is inserted and linked to the vector for introduction into the host bacterium. As the host bacterium is proliferated, the vector DNA linked to the target DNA fragment is replicated and distributed to each daughter cell hand in hand the division of bacteria to maintain the target DNA fragment from generation to generation, and a plasmid and a phage chromosome are usually used.

As used herein, the "plasmid" is a generic term for a gene which is stably maintained and transmitted to progeny throughout generations in cells, which exists independently of chromosomes and autonomously propagates, but DNA included in mitochondria or chloroplasts of eukaryotic cells is generally called organelle DNA, and is distinguished from the plasmid. The presence of the gene is not always essential for normal cell survival, but in bacterial cells, the gene has functions such as conjugal transfer (F factor), resistance to antibiotics (R factor), and synthesis of antibacterial materials (bacteriocin) (colicin factor). In addition, there are also those that transform plant cells into tumors, such as the Ti plasmids present in soil bacteria, and various forms, such as those which are co-delivered when deliverability factors coexist with each other even if they do not have their own delivery mechanism or those which are not delivered even though they co-exist have been discovered. The plasmid is often used as a vector in tissue conversion DNA experiments (gene manipulation techniques).

As used herein, the "promoter" refers to a genomic region linked to the upstream part of a structural gene, and serves to regulate the structural gene linked thereto to be transcribed into mRNA. The promoter is activated by binding multiple general transcription factors, and includes a TATA box that universally regulates gene expression, a CAAT box region, a region which affects gene expression in response to external stimuli, an enhancer which promotes the expression of almost all genes regardless of position and direction, and the like. Since the proteins required for basic metabolism of an organism have to maintain a constant concentration in cells, the promoters linked to these genes are always activated only by the action of general transcription factors. In contrast, proteins, which have no role in normal times and are required to function only under special circumstances, are linked to a tissue specific promoter or inducible promoter which induces expression of the corresponding structural gene. That is, the tissue specific promoter is activated by the binding of an activated specific transcription factor during the development process of an organism, and the inducible promoter is activated by the binding of a specific transcription factor activated by an external stimulus from the surrounding environmental factors.

As used herein, the "operator" is also called an operator gene, and serves to regulate the expression of a specific gene during the gene regulation of bacteria. The operator is primarily located next to a structural gene and serves to regulate the expression or non-expression of the required gene under specific circumstances.

As used herein, the "recombinant vector" refers to a gene construct including an essential regulatory element operably linked to express a gene insert as a vector capable of expressing a target protein or a target RNA in a host cell, and refers to plasmids, viruses or other vehicles known in the art, in which gene base sequences encoding a promoter and a target protein operably linked to the promoter can be inserted or introduced.

In addition, the present invention provides a transformant transformed with a recombinant vector prepared by the present invention.

As used herein, the "transformation" means that the trait of an individual or a cell is genetically modified by DNA, which is a genetic material given from the outside.

Another aspect of the present invention provides a method for producing a transformed protein, the method comprising: a) constructing a recombinant vector of the present invention; b) transforming *E. coli* with the recombinant vector; and c) culturing the *E. coli* in a medium.

In an exemplary embodiment of the present invention, a plasmid was constructed using a pGE vector and a reference DNA sequence in which thymosin beta 4 (TB4) is linked to the carboxyl terminus (C-term) of a disulfide bond isomerase signal peptide (DsbC-SP) which is a signal peptide (see Example 1).

In another exemplary embodiment of the present invention, transformed *E. coli* strains BL21 and DH5$\alpha$ were constructed using the constructed plasmid (see Example 2), and the growth of the transformed *E. coli* strain BL21 was confirmed (see Example 3).

In still another exemplary embodiment of the present invention, it was confirmed that TB4 was expressed in the transformed *E. coli* strain (see Example 4).

Hereinafter, preferred examples for helping the understanding of the present invention will be suggested. However, the following Examples are provided only to more easily understand the present invention, and the contents of the present invention are not limited by the following Examples.

## Example 1. Construction of plasmid

In order to synthesize a plasmid which allows thymosin beta (TB4) to be expressed, by using a pGE vector and a reference DNA sequence in which TB4 was linked to the carboxyl terminus (C-term) of a disulfide bond isomerase signal peptide (DsbC-SP) which is a signal peptide, a plasmid in which the DNA sequence was inserted into a vector was synthesized.

The base sequences for plasmid synthesis are shown in the following Table 1.

[Table 1]

| Base sequence | | SEQ ID NO |
|---|---|---|
| **DsbC-SP** | ATGAAGAAAGGTTTTATGTTATTTACCTTGTTGGCAGCGTTTTCAGGCTTTGTTCAGGCT | SEQ ID NO: 1 |
| **TB4** | TCCGACAAACCCGATATGGCTGAGATCGAGAAATTCGATAAGTCGAAACTGAAGAAGACAGAGACGCAAGAGAAAAATCCACTGCCTTCCAAAGAAACGATTGAACAGGAGAAGCAAGCAGGCGAATCGTAA | SEQ ID NO: 2 |
| **EcoRI** | GAATTC | SEQ ID NO: 3 |
| **Tac Promoter** | TTGACAATTAATCATCGGCTCGTATAATG | SEQ ID NO: 4 |
| **Lac Operator** | TTGTGAGCGGATAACAA | SEQ ID NO: 5 |
| **XhoI** | CTCGAG | SEQ ID NO: 6 |
| **DsbC-SP TB4** | ATGAAGAAAGGTTTTATGTTATTTACCTTGTTGGCAGCGTTTTCAGGCTTTGTTCAGGCTTCCGACAAACCCGATATGGCTGAGATCGAGAAATTCGATAAGTCGAAACTGAAGAAGACAGAGACGCAAGAGAAAAATCCACTGCCTTCCAAAGAAACGATTGAACAGGAGAAGCAAGCAGGCGAATCGTAA | SEQ ID NO: 7 |

As a result of confirming that the DNA sequence of the synthesized plasmid and the reference DNA sequence were aligned and matched (Http://Multalin.toulouse.inra.fr/), the two sequences were 100% identical, as illustrated in FIG. 1.

Further, a vector map as illustrated in FIG. 2 could be confirmed, and the synthesized plasmid was named "DsbC-SP TB4".

## Example 2. Construction of transformed *E. coli* strain

A transformed *E. coli* strain was constructed by performing the following experiment using a plasmid constructed by the method in Example 1.

### <2-1> Preparation of LB medium

In order to prepare a Luria Bertani media broth (LB broth), 800 mL of tertiary water was put into a 2 L beaker, 25.03 g of an LB broth was measured, the LB broth was put into the 2 L beaker, and then the resulting mixture was stirred until it was completely dissolved. And then, the final volume was adjusted to 1 L by adding tertiary water thereto, the resulting product was transferred to a 2 L bottle, and the bottle was sterilized under the conditions of 121°C and 30 minutes.

### 2-2. Preparation of LB agar plate supplemented with kanamycin

In order to prepare an LB agar plate, 300 mL of tertiary water was put into a 2 L beaker, 19.98 g of LB agar was put into the 2 L beaker, and the resulting mixture was stirred until it was completely dissolved. And then, the final volume was adjusted to 500 mL by adding tertiary water thereto, the resulting product was transferred to a 2 L bottle, and the bottle was sterilized under the conditions of 121°C and 30 minutes. And then, the bottle was taken out from the autoclave when the temperature of the autoclave was 40 to 50°C and transferred to a clean bench, and 500 μL of kanamycin (stock concentration of 50 mg/mL) was added thereto until the concentration became 50 μg/mL. About 20 mL of the LB agar solution was dispensed into a petri dish, the petri dish was dried in a clean bench until the LB agar of the petri dish was solidified, and then the fully dried petri dish was wrapped in plastic wrap and stored in a refrigerator (2 to 8°C) for up to 1 month.

### 2-3. Construction of transformed *E. coli* strain for protein expression

First, in order to construct a transformed *E. coli* strain BL21 (BL21/DsbC-SP TB4) for the expression of a target protein using the plasmid (DsbC-SP TB4) synthesized by the method in Example 1, transformation was performed according to the user protocol TB009 (Novagen), and the transformed strain was spread on an LB agar plate supplemented with 50 μg/mL kanamycin and cultured in an incubator set at 37°C overnight (14 hours).

It was confirmed that in the transformed *E. coli* BL21, colonies were formed in the LB agar plate supplemented with kanamycin (50 μg/mL), and a single colony of BL21/DsbC-SP TB4 was collected by loop and needle. The LB agar plate supplemented with kanamycin (50 μg/mL) was sealed with Parafilm and stored in a refrigerator (2 to 8°C) for up to 1 month.

And then, the collected BL21/DsbC-SP TB4 was inoculated into a 14 mL round-bottom tube containing 4 mL of an LB broth, cultured in a shaking incubator set at 37°C and 200 rpm overnight (14 hours), 200 μL of a culture solution was inoculated into a 250 ml flask containing 100 ml of the LB broth, and then the culture was terminated when $OD_{600}$ = approximately 0.2 by measuring the $OD_{600}$ value at intervals of 30 minutes. The *E. coli* strain was recovered by centrifugation at 4,000 rpm for 15 minutes, and the volume at which $OD_{600}$ = 1.0 was calculated according to the following calculation equation.

$$(\text{Volume at which } OD_{600} = 1.0) = (\text{Volume at the end of culture}) \times (OD_{600} \text{ value at the end time point of culture})$$

The pellet was resuspended by putting the LB broth containing sterilized glycerol so as to have a volume of 15% at a volume calculated by the above equation, and then 200 μL each of the suspension was aliquoted into sterilized microtubes, and the microtubes were stored in a deep freezer (-80°C to -70°C) or an $LN_2$ tank (-196°C).

And then, as a result of constructing a transformed *E. coli* strain DH5α (DH5α/DsbC-SP TB4) in order to mass-produce plasmid DNA using a DsbC-SP TB4 plasmid by the same method, a plate in which colonies were formed was obtained.

The DH5α/DsbC-SP TB4 constructed as described above was subjected to the same process for the single colony of the BL21/DsbC-SP TB4 and stored in a deep freezer (-80°C to -70°C) or an $LN_2$ tank (-196°C).

## Example 3. Confirmation of growth of constructed *E. coli* strain

In order to confirm the growth of the *E. coli* BL21 constructed by the method in Example 2, 20 ml of the LB Broth and 20 μl of the kanamycin stock (50 mg/ml) were placed in a 50 mL conical tube. After the DsbC-SP TB4 1 vial stored in the deep freezer was taken out, the vial was thawed in a water bath set at 37°C for 2 to 3 minutes. The thawed cells were inoculated into the 50 ml conical tube containing the LB broth and kanamycin, and cultured in a shaking incubator at 37°C and 200 rpm for 3 hours. And then, 2.5 ml of the culture solution was inoculated into a baffled Erlenmeyer flask containing 250 ml of the LB broth, and 1 mL of the culture solution was sampled (sampling) every hour after the inoculation. The $OD_{600}$ value of the extracted culture solution was measured by a UV spectrophotometer, and when the measured $OD_{600}$ value was 0.8 or higher, the culture solution was diluted and measured again.

As a result, as illustrated in FIG. 3, it could be confirmed that the $OD_{600}$ value had increased to about 2.5 after 6 hours of culture of the transformed *E. coli* strain BL21 (BL21/DsbC-SP TB4) using the synthesized plasmid (DsbC-SP TB4).

## Example 4. Confirmation of expression of TB4 in constructed *E. coli* strain

### 4-1. Confirmation of expression of DsbC-SP TB4

In order to confirm the expression of a thymosin beta 4 (TB4) protein in the *E. coli* strain constructed by the method in Example 2, the DsbC-SP TB4 cell stock was thawed in 20 mL of an LB medium supplemented with kanamycin (50 μg/mL). And then, after the cell stock was cultured in a shaking incubator set at 37°C and 200 rpm for 3 hours (a primary seed culture solution), the primary seed culture solution was diluted 1/10,000 in 20 mL of an LB medium containing kanamycin (50 μg/mL), and then cultured overnight (a secondary seed culture solution). 2.5 mL of the secondary seed culture solution was inoculated into 250 mL of LB and cultured again in a shaking incubator set at 37°C and 200 rpm, and the $OD_{600}$ value was measured every 1 hour.

When the measured $OD_{600}$ value reached 0.6, IPTG was added thereto for induction so as to become 1.0 mM, and the culture was terminated at 3 hours after the induction. Cells were recovered by centrifuging 10 mL of the culture solution at 4,000 rpm for 30 minutes, and the pellet was crushed with B-PER according to User Guide: B-PER Bacterial Protein Extraction Reagent, and then centrifuged at 15,000 rpm for 10 minutes to isolate only the supernatant. After a sample for SDS-PAGE was produced by mixing the isolated supernatant with a 4x Laemmli sample buffer at a ratio of 1 : 3 and loaded onto a gel, the sample was subjected to electrophoresis at 200 V for 30 minutes, Coomassie-stained for 30 minutes, and then destained. Thereafter, a target protein band was analyzed using Gel-doc.

When the expression of the protein was induced, the growth degree of BL21/DsbC-SP TB4 is as illustrated in FIG. 4.

**4-2. N-term sequencing of DsbC-SP TB4**

The DsbC-SP TB4 cell stock was thawed in 20 mL of an LB medium supplemented with kanamycin (50 μg/mL), and cultured in a shaking incubator set at 37°C and 200 rpm for 3 hours (a primary seed culture solution). And then, the primary seed culture solution was diluted 1/10,000 in 20 mL of an LB medium supplemented with kanamycin (50 μg/mL), and then cultured overnight (a secondary seed culture solution). 2.5 mL of the secondary seed culture solution was inoculated into 250 mL of LB and cultured again in a shaking incubator set at 37°C and 200 rpm, and the $OD_{600}$ value was measured every 1 hour.

When the measured $OD_{600}$ value reached 0.6, IPTG was added thereto for induction so as to become 1.0 mM, and the culture was terminated at 3 hours after the induction. Cells were recovered by centrifuging 10 mL of the culture solution at 4,000 rpm for 30 minutes, and the pellet was crushed with B-PER according to User Guide: B-PER Bacterial Protein Extraction Reagent, and then centrifuged at 15,000 rpm for 10 minutes to isolate only the supernatant. After a sample for SDS-PAGE was produced by mixing the isolated supernatant with a 4x Laemmli sample buffer at a ratio of 1 : 3 and loaded onto a gel, the sample was subjected to electrophoresis at 200 V for 30 minutes. The sample was transferred to a PVDF membrane, and a band of a transformed target protein was confirmed by staining the membrane with Ponceau.

As a result, as illustrated in FIG. 5, it was confirmed that an induced band appeared at the same position as the TB4 standard, and each lane description is as shown in the following Table 2.

[Table 2]

| M | Marker |
|---|---|
| 1 | Gly-TB4(Y-20150303) |
| 2 | Induction at OD 0.6 with IPTG 1.0mM |

Further, as a result of analyzing the sequence with respect to 10 amino acids of the protein N-term corresponding to the expected target band size (5 to 6 kDa) confirmed above, as shown in the following Table 3, the sequence matched that of WT (wild type)_TB4.

[Table 3]

| Sample No 1 : 20160527_5kDa | |
|---|---|
| Prediction | S-D-K-P-D-M-A-E-I-E |
| Result | S-D-K-P-D-M-A-E-I-E |
| N-term sequencing result | N-term sequence of Expected Target Size Band matched that of TB4 |

Meanwhile, the amino acid sequence of WT_TB4 is as shown in the following Table 4, and in particular, the part expressed in **bold** type refers to the sequence of 10 N-term amino acids.

[Table 4]

| Amino acid sequence of WT_TB4 | SEQ ID NO |
|---|---|
| **SDKPDMAEIE**KFDKSKLKKTETQEKNPLPSKETIEQEKQAGES | SEQ ID NO: 8 |

From the above, it can be seen that the *E. coli* strain constructed by the method of Example 2 is a strain which is transformed with a plasmid having a sequence of WT_TB4 and expresses WT_TB4.

For reference, the base sequence and amino acid sequence of the disulfide bond isomerase signal peptide of the present invention are shown in the following Table 5, but are not limited thereto.

[Table 5]

| **Sequence name** | **Classification** | **Sequence** | **SEQ ID NO** |
|---|---|---|---|
| **DsbC-SP1** | Base sequence | 5'-atg aag aaa ggt ttt atg tta ttt acc ttg ttg gca gcg ttt tca ggc ttt gtt cag gct-3' | SEQ ID NO: 9 |
| | Amino acid sequence | $NH_2$-MKKGFMLFTLLAAFSGFVQA-COOH | SEQ ID NO: 10 |
| **DsbC-SP2** | Base sequence | 5'-atg aaa aaa att att aag gca tcg gta tta ctt ctt tca tta agt acc gcc ttc acg atg aat gcc gag 3' | SEQ ID NO: 11 |
| | Amino acid sequence | $NH_2$-MKKIIKASVLLLSLSTAFTMNAE-COOH | SEQ ID NO: 12 |
| **DsbC-SP3** | Base sequence | 5'- atg cgg aca aaa tta ctt ggg gcg ctg atg gtg ttc ggg att att acc ggc acg gct cat gcg tca-3' | SEQ ID NO: 13 |
| | Amino acid sequence | $NH_2$-MRTKLLGALMVFGIITGTAHAS-COOH | SEQ ID NO: 14 |
| **DsbA-SP** | Base sequence | 5'-atg aaa aag att tgg ctg gcg ctg gct ggt tta gtt tta gcg ttt agc gca tcg gcg gcg-3' | SEQ ID NO: 15 |
| | Amino acid sequence | $NH_2$-NMKIWLALAGLVLF SASAA-COOH | SEQ ID NO: 16 |

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

[Industrial Applicability]

When a recombinant vector comprising a disulfide bond isomerase signal peptide is used, it is possible to produce a protein whose amino acid sequence does not start with methionine through an *E. coli* expression system, and accordingly, there is an advantage in that the recombinant vector does not need additional processes such as a protease treatment for commercial availability. In particular, although thymosin beta 4 has been highly available as a therapeutic agent for various diseases for a while, thymosin beta 4 has a problem in that when thymosin beta 4 is expressed in a host (host strain), thymosin beta 4 is degraded by intracellular proteases and cannot be produced. The present invention enables the thymosin beta 4 to be produced through an *E. coli* expression system, and thus to be usefully utilized in the development of a therapeutic agent for alopecia as well as ischemic heart disease, corneal injury, ophthalmic diseases, and epidermolysis bullosa, and further, the *E. coli* expression system of the present invention can be applied to production of various therapeutic proteins such as a parathyroid hormone, a human growth hormone, and a granulocyte-colony stimulating factor (GCSF) used for the treatment of osteoporosis, and thus is expected to be widely used as a useful technique.

## Claims

1. A recombinant vector comprising a base sequence encoding a disulfide bond isomerase signal peptide.

2. The recombinant vector of claim 1, wherein the disulfide bond isomerase signal peptide comprises any one amino acid sequence selected from the group consisting of SEQ ID NOS: 10, 12, 14, and 16.

3. The recombinant vector of claim 1, further comprising a base sequence encoding thymosin beta 4.

4. The recombinant vector of claim 3, wherein the thymosin beta 4 is encoded by a base sequence of SEQ ID NO: 2.

5. The recombinant vector of claim 1, further comprising one or more selected from the group consisting of EcoRI represented by SEQ ID NO: 3, a Tac promoter represented by SEQ ID NO: 4, a Lac operator represented by SEQ ID NO: 5, and XhoI represented by SEQ ID NO: 6.

6. A recombinant vector comprising a base sequence encoding a disulfide bond isomerase signal peptide and a base sequence encoding thymosin beta 4.

7. The recombinant vector of claim 6, wherein the disulfide bond isomerase signal peptide comprises any one amino acid sequence selected from the group consisting of SEQ ID NOS: 10, 12, 14, and 16.

8. The recombinant vector of claim 6, wherein the thymosin beta 4 is encoded by a base sequence of SEQ ID NO: 2.

9. The recombinant vector of claim 6, further comprising one or more selected from the group consisting of EcoRI represented by SEQ ID NO: 3, a Tac promoter represented by SEQ ID NO: 4, a Lac operator represented by SEQ ID NO: 5, and XhoI represented by SEQ ID NO: 6.

10. A transformant transformed with the recombinant vector of any one of claims 1 to 9.

11. The transformant of claim 10, wherein the transformant is *E. coli.*

12. A method for producing a thymosin beta 4 protein, the method comprising: a) constructing the recombinant vector of any one of claims 1 to 9:

    b) transforming *E. coli* with the recombinant vector; and
    c) culturing the *E. coli* in a medium,

    wherein the thymosin beta 4 protein has an amino sequence which does not start with methionine.

FIG. 1

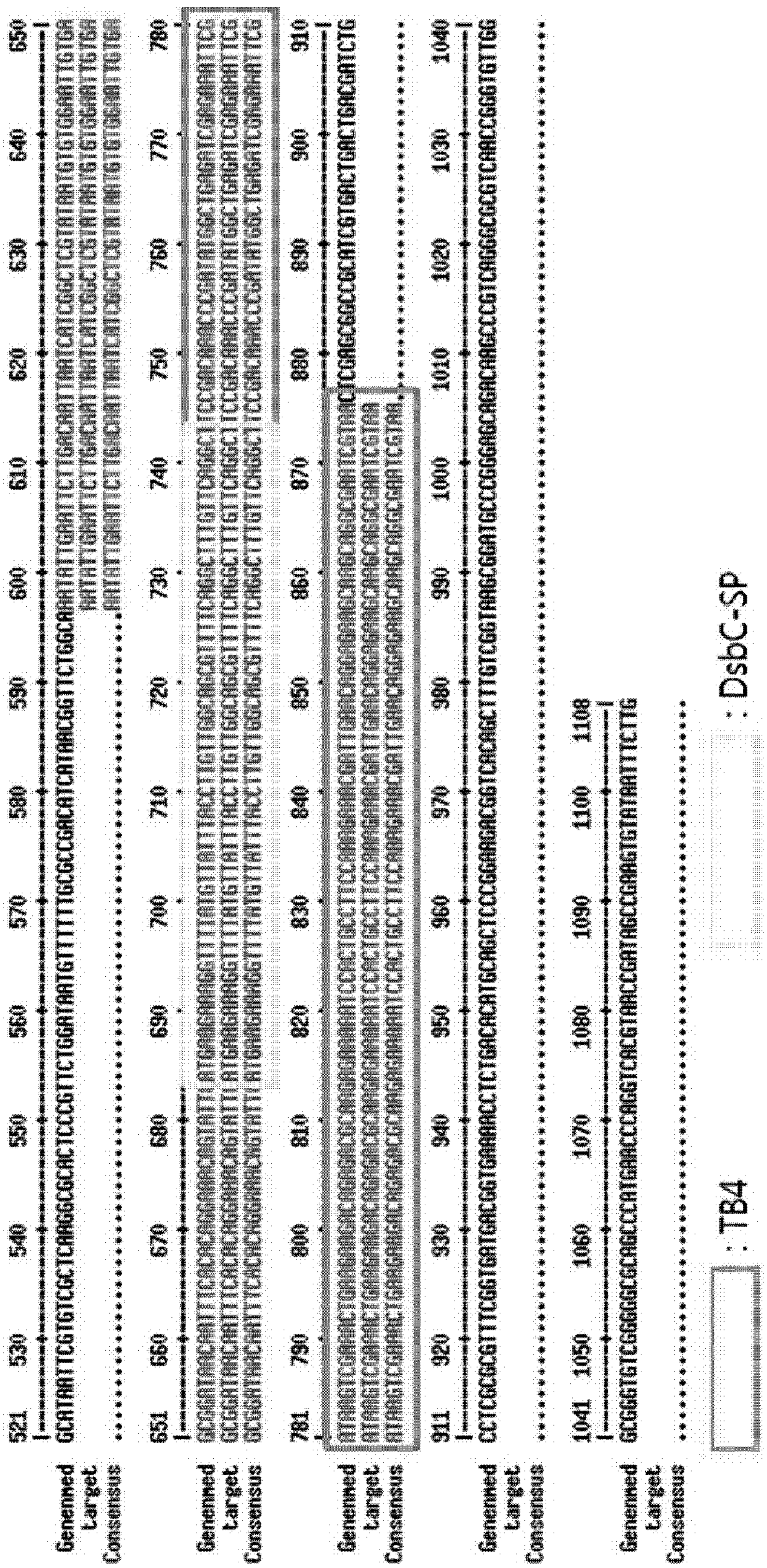

FIG. 2

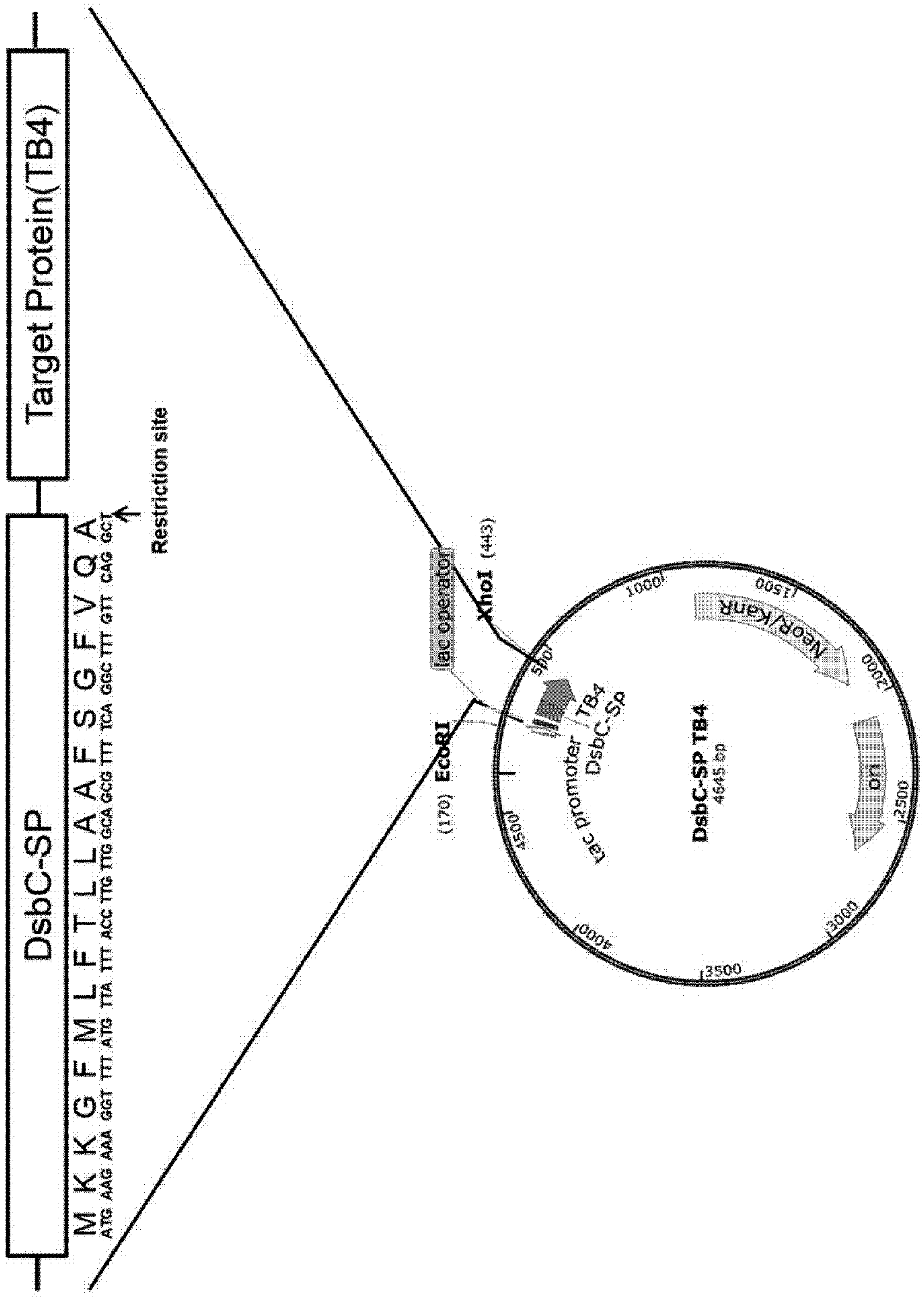
DsbC-SP
Target Protein(TB4)
M K K G F M L F T L L A A F S G F V Q A
ATG AAG AAA GGT TTT ATG TTA TTT ACC TTG TTG GCA GCG TTT TCA GGC TTT GTT CAG GCT
Restriction site
lac operator
XhoI (443)
EcoRI (170)
tac promoter
DsbC-SP
TB4
NeoR/KanR
ori
DsbC-SP TB4
4645 bp
500
1000
1500
2000
2500
3000
3500
4000
4500

FIG. 3

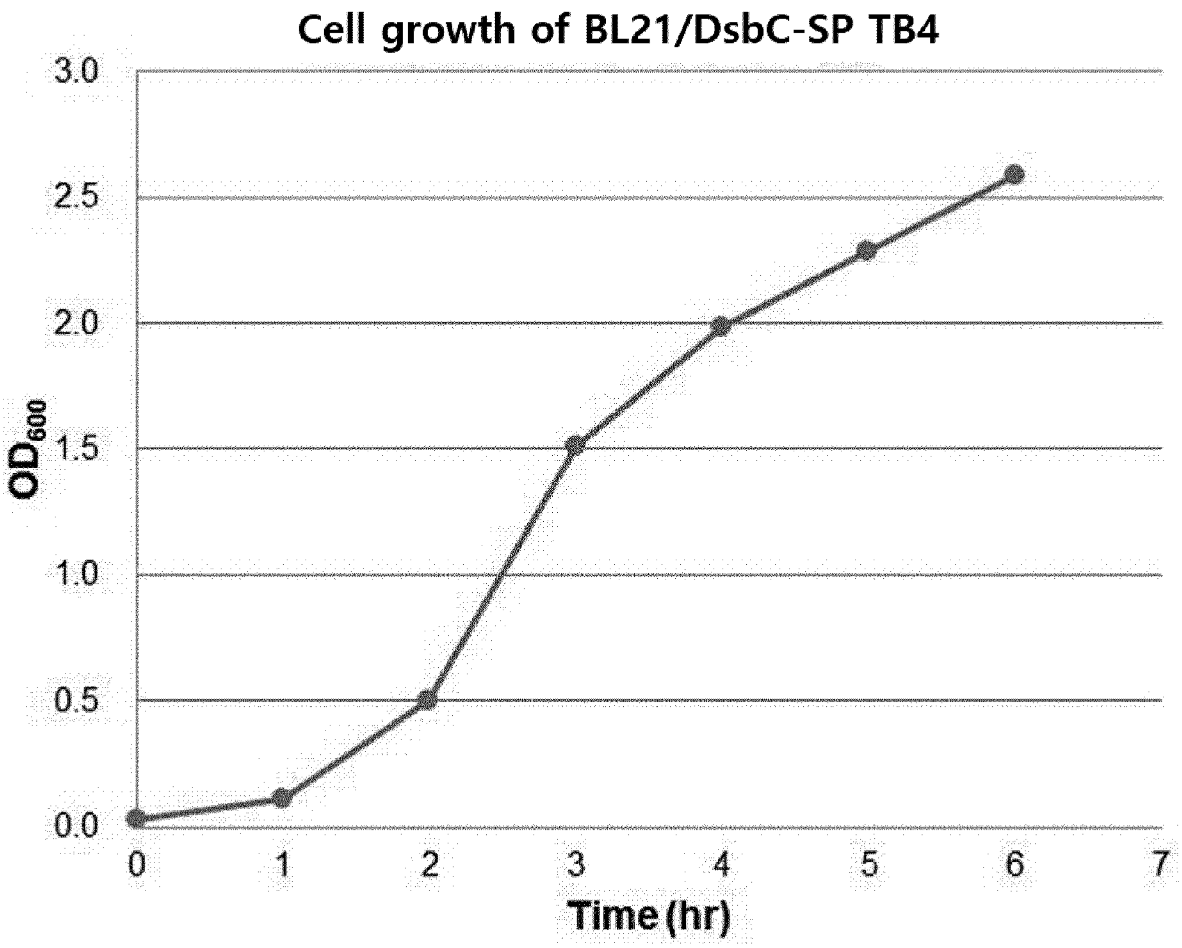
Cell growth of BL21/DsbC-SP TB4
3.0
2.5
2.0
1.5
1.0
0.5
0.0
OD600
0
1
2
3
4
5
6
7
Time (hr)

FIG. 4

Cell growth of BL21/DsbC-SP TB4 during induction of protein expression

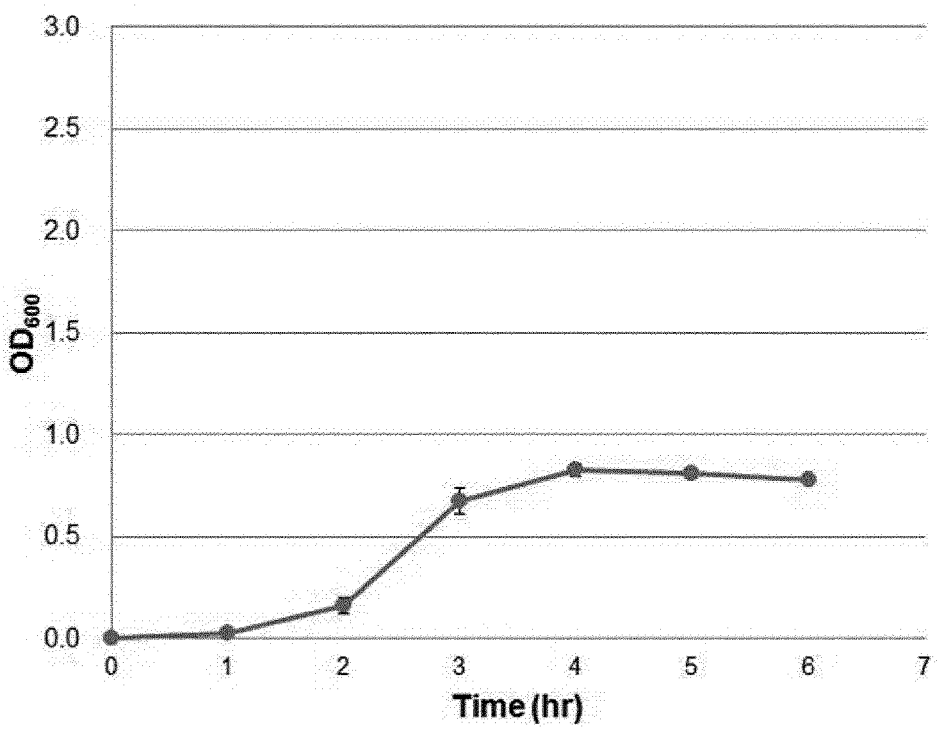

FIG. 5

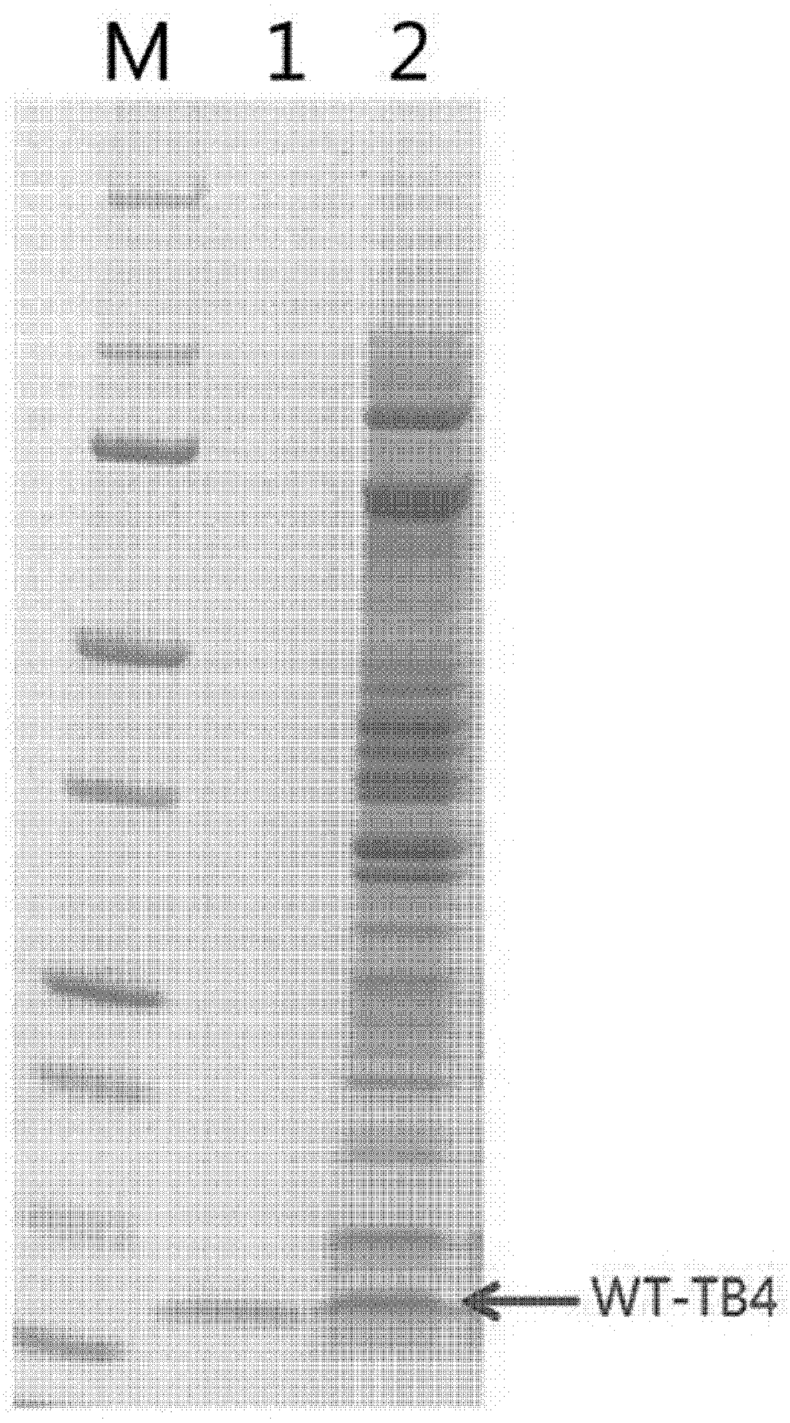

## INTERNATIONAL SEARCH REPORT

International application No.
PCT/KR2019/001055

A. CLASSIFICATION OF SUBJECT MATTER

*C12N 15/70(2006.01)i, C07K 14/575(2006.01)i, C07K 7/08(2006.01)i, C07K 14/00(2006.01)i*

According to International Patent Classification (IPC) or to both national classification and IPC

B. FIELDS SEARCHED

Minimum documentation searched (classification system followed by classification symbols)
C12N 15/70; C07K 16/00; C12N 15/62; C07K 14/575; C07K 7/08; C07K 14/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: disulfide bond isomerase signal peptide, thymosin beta 4, recombination vector

C. DOCUMENTS CONSIDERED TO BE RELEVANT

| Category* | Citation of document, with indication, where appropriate, of the relevant passages | Relevant to claim No. |
|---|---|---|
| Y | WO 2008-094986 A2 (DOW GLOBAL TECHNOLOGIES, INC. et al.) 07 August 2008<br>See abstract and claims 6, 28, 35, 38. | 1-12 |
| Y | KR 10-2007-0021079 A (HANMI PHARM. CO., LTD.) 22 February 2007<br>See abstract, claim 1 and paragraphs [0028], [0048]. | 1-12 |
| Y | NCBI. genbank accession number: WP_042104759.1. bifunctional protein-disulfide isomerase/oxidoreductase DsbC. partial [Escherichia coli]. 04 May 2016<br>See the entire document. | 2,7 |
| Y | NCBI. genbank accession number: EIC48796.1. DsbC, partial [Salmonella enterica subsp. enterica serovar Heidelberg str. 41565]. 09 April 2012<br>See the entire document. | 2,7 |
| Y | NCBI. genbank accession number: PAT70937.1. disulfide isomerase, partial [Klebsiella pneumoniae]. 05 September 2017<br>See the entire document. | 2,7 |
| Y | NCBI. genbank accession number: AAA82614.1. thiol:disulfide interchange protein DsbA mutant PH31/32stopV [Escherichia coli]. 02 December 1995<br>See the entire document. | 2,7 |

☒ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
27 MAY 2019 (27.05.2019)

Date of mailing of the international search report
27 MAY 2019 (27.05.2019)

Name and mailing address of the ISA/KR
Korean Intellectual Property Office
Government Complex Daejeon Building 4, 189, Cheongsa-ro, Seo-gu, Daejeon, 35208, Republic of Korea
Facsimile No. +82-42-481-8578

Authorized officer

Telephone No.

**INTERNATIONAL SEARCH REPORT**

International application No.
**PCT/KR2019/001055**

| C (Continuation). DOCUMENTS CONSIDERED TO BE RELEVANT | | |
|---|---|---|
| Category* | Citation of document, with indication, where appropriate, of the relevant passages | Relevant to claim No. |
| Y | NCBI. genbank accession number: XM_009234588.1. PREDICTED: Pongo abelii thymosin beta 4, X-linked (TMSB4X), transcript variant X1. mRNA. 23 September 2014 See the entire document. | 3-4,6-9 |

Form PCT/ISA/210 (continuation of second sheet) (January 2015)

**INTERNATIONAL SEARCH REPORT**
Information on patent family members

International application No.
**PCT/KR2019/001055**

| Patent document cited in search report | Publication date | Patent family member | Publication date |
|---|---|---|---|
| WO 2008-094986 A2 | 07/08/2008 | AU 2008-210538 A1 | 07/08/2008 |
| | | AU 2008-210538 B2 | 06/06/2013 |
| | | BR PI0807834 A2 | 22/07/2014 |
| | | CA 2677179 A1 | 07/08/2008 |
| | | CA 2677179 C | 16/02/2016 |
| | | CN 101641441 A | 03/02/2010 |
| | | CN 102517280 A | 27/06/2012 |
| | | EP 2108047 A2 | 14/10/2009 |
| | | EP 2108047 B1 | 24/10/2012 |
| | | EP 2468869 A1 | 27/06/2012 |
| | | EP 2468869 B1 | 18/03/2015 |
| | | JP 2010-517532 A | 27/05/2010 |
| | | JP 2015-144608 A | 13/08/2015 |
| | | JP 2018-102305 A | 05/07/2018 |
| | | JP 5714230 B2 | 07/05/2015 |
| | | JP 6495690 B2 | 03/04/2019 |
| | | KR 10-1491867 B1 | 10/02/2015 |
| | | KR 10-2009-0114422 A | 03/11/2009 |
| | | US 2008-0193974 A1 | 14/08/2008 |
| | | US 2010-0048864 A1 | 25/02/2010 |
| | | US 2011-0020868 A1 | 27/01/2011 |
| | | US 7618799 B2 | 17/11/2009 |
| | | US 7833752 B2 | 16/11/2010 |
| | | WO 2008-094986 A3 | 20/11/2008 |
| | | WO 2008-094986 A8 | 07/08/2008 |
| KR 10-2007-0021079 A | 22/02/2007 | AU 2006-280587 A1 | 22/02/2007 |
| | | AU 2006-280587 B2 | 24/02/2011 |
| | | CA 2619771 A1 | 22/02/2007 |
| | | CA 2619771 C | 22/05/2012 |
| | | CN 101258164 A | 03/09/2008 |
| | | CN 101258164 B | 01/05/2013 |
| | | EP 1928910 A1 | 11/06/2008 |
| | | EP 1928910 B1 | 15/01/2014 |
| | | JP 2009-513110 A | 02/04/2009 |
| | | JP 2014-110816 A | 19/06/2014 |
| | | JP 5846712 B2 | 20/01/2016 |
| | | US 2008-0293106 A1 | 27/11/2008 |
| | | US 7968316 B2 | 28/06/2011 |
| | | WO 2007-021129 A1 | 22/02/2007 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020180010055

<110> Huons Co., Ltd.
Huons Global Co., Ltd.

<120> Recombination vector comprising Disulfide Bond Isomerase signal peptide and use thereof

<130> MPCT18-091

<150> KR 10-2018-0010055
<151> 2018-01-26

<160> 16

<170> KoPatentIn 3.0

<210> 1
<211> 60
<212> DNA
<213> Artificial Sequence

<220>
<223> DsbC-SP

<400> 1
atgaagaaag gttttatgtt atttaccttg ttggcagcgt tttcaggctt tgttcaggct 60

60

<210> 2
<211> 132
<212> DNA
<213> Artificial Sequence

<220>
<223> TB4

<400> 2
tccgacaaac ccgatatggc tgagatcgag aaattcgata agtcgaaact gaagaagaca 60

gagacgcaag agaaaaatcc actgccttcc aaagaaacga ttgaacagga gaagcaagca 120

ggcgaatcgt aa 132

<210> 3
<211> 6
<212> DNA
<213> Artificial Sequence

<220>
<223> EcoRI

<400> 3
gaattc 6

<210> 4
<211> 29
<212> DNA

<213> Artificial Sequence

<220>
<223> Tac Promoter

<400> 4
ttgacaatta atcatcggct cgtataatg 29

<210> 5
<211> 17
<212> DNA
<213> Artificial Sequence

<220>
<223> Lac Operator

<400> 5
ttgtgagcgg ataacaa 17

<210> 6
<211> 6
<212> DNA
<213> Artificial Sequence

<220>
<223> XhoI

<400> 6
ctcgag 6

<210> 7
<211> 192
<212> DNA
<213> Artificial Sequence

<220>
<223> DsbC-SP TB4

<400> 7
atgaagaaag gttttatgtt atttaccttg ttggcagcgt tttcaggctt tgttcaggct 60

tccgacaaac ccgatatggc tgagatcgag aaattcgata agtcgaaact gaagaagaca 120

gagacgcaag agaaaaatcc actgccttcc aaagaaacga ttgaacagga gaagcaagca 180

ggcgaatcgt aa 192

<210> 8
<211> 43
<212> PRT
<213> Artificial Sequence

<220>
<223> WT_TB4

<400> 8
Ser Asp Lys Pro Asp Met Ala Glu Ile Glu Lys Phe Asp Lys Ser Lys
1 5 10 15

Leu Lys Lys Thr Glu Thr Gln Glu Lys Asn Pro Leu Pro Ser Lys Glu
20 25 30

Thr Ile Glu Gln Glu Lys Gln Ala Gly Glu Ser
35 40

<210> 9
<211> 60
<212> DNA
<213> Artificial Sequence

<220>
<223> DsbC-SP1

<400> 9
atgaagaaag gttttatgtt atttaccttg ttggcagcgt tttcaggctt tgttcaggct 60

60

<210> 10
<211> 20
<212> PRT
<213> Artificial Sequence

<220>
<223> DsbC-SP1

<400> 10
Met Lys Lys Gly Phe Met Leu Phe Thr Leu Leu Ala Ala Phe Ser Gly
1 5 10 15

Phe Val Gln Ala
20

<210> 11
<211> 69
<212> DNA
<213> Artificial Sequence

<220>
<223> DsbC-SP2

<400> 11
atgaaaaaaa ttattaaggc atcggtatta cttctttcat taagtaccgc cttcacgatg 60

aatgccgag 69

<210> 12
<211> 23
<212> PRT
<213> Artificial Sequence

<220>
<223> DsbC-SP2

<400> 12
Met Lys Lys Ile Ile Lys Ala Ser Val Leu Leu Leu Ser Leu Ser Thr
1 5 10 15

Ala Phe Thr Met Asn Ala Glu
20

<210> 13
<211> 66
<212> DNA
<213> Artificial Sequence

<220>
<223> DsbC-SP3

<400> 13
atgcggacaa aattacttgg ggcgctgatg gtgttcggga ttattaccgg cacggctcat 60

gcgtca 66

<210> 14
<211> 22
<212> PRT
<213> Artificial Sequence

<220>
<223> DsbC-SP3

<400> 14
Met Arg Thr Lys Leu Leu Gly Ala Leu Met Val Phe Gly Ile Ile Thr
1 5 10 15

Gly Thr Ala His Ala Ser
20

<210> 15
<211> 60
<212> DNA
<213> Artificial Sequence

<220>
<223> DsbA-SP

<400> 15
atgaaaaaga tttggctggc gctggctggt ttagttttag cgtttagcgc atcggcggcg 60

60

<210> 16
<211> 19
<212> PRT

```
<213>  Artificial Sequence

<220>
<223>  DsbA-SP


<400>  16
Met Lys Lys Ile Trp Leu Ala Leu Ala Gly Leu Val Leu Phe Ser Ala
  1               5                  10                  15

Ser Ala Ala
```